# EUROPEAN PATENT APPLICATION

(11) **EP 4 483 913 A1**
(43) Date of publication of application: **01.01.2025**
(21) Application number: 22933133.5
(22) Date of filing: 30.11.2022
(51) Int. Cl.: A61L 31/16, A61L 31/08

(54) **DRUG COATING MEDICAL INSTRUMENT, PREPARATION METHOD THEREFOR AND USE THEREOF, AND DRUG COATING AND USE THEREOF**

(30) Priority: 21.03.2022 CN 202210276877
(71) Applicant: Mitrassist Lifesciences Limited, Shanghai 201807 (CN)
(72) Inventor: ZHONG, Wei, Shanghai 201807 (CN)
(74) Representative: Berggren Oy
(86) International application number: PCT/CN2022/135610
(87) International publication number: WO 2023/179082

(57) **Abstract**

The present invention relates to the technical field of medical instruments, in particular to a drug coating medical instrument, a preparation method therefor and use thereof, and a drug coating and use thereof. The drug coating medical instrument comprises a carrier and a drug coating partially or completely covering the carrier, wherein the drug coating is mainly obtained by coating a coating liquid comprising a macrolide drug and a dispersant; the dispersant comprises water and/or an organic solvent, and the organic solvent includes at least one of ethanol, acetone, dichloromethane, 1,4-dioxane, tetrahydrofuran, and toluene. The drug coating medical instrument provided by the present invention can transfer enough drugs to a blood vessel wall in the process of being in contact with the blood vessel wall, and a certain drug concentration is still maintained in the blood vessel wall tissue for a relatively long time after the drug coating medical instrument is withdrawn.

## Description

### CROSS-REFERENCE TO RELATED APPLICATION

The present invention claims the priority to the Chinese patent application with the filing No. CN202210276877.8 filed with the Chinese Patent Office on March 21, 2022, and entitled "DRUG COATING MEDICAL INSTRUMENT, PREPARATION METHOD THEREFOR AND USE THEREOF, AND DRUG COATING AND USE THEREOF", the contents of which are incorporated herein by reference in entirety.

### TECHNICAL FIELD

The present invention relates to the technical field of medical devices, and specifically to a drug coat medical device (i.e., drug coating medical instrument), a preparation method therefor and use thereof, and a drug coat (i.e., drug coating) and use thereof.

### BACKGROUND ART

Macrolide drugs such as sirolimus, everolimus, tacrolimus and zotarolimus have been proved to have good anti-proliferation effects, can be loaded on a surface of an intravascular implantable device, and can play a role in inhibiting proliferation of vascular smooth muscle cells and reducing restenosis through slow release within a certain period of time. However, existence of an intravascular implant also will bring about a series of negative effects. Therefore, it is expected that, on the one hand, drugs can be delivered to a lesion blood vessel while opening the occluded or stenosed lesion blood vessel; and meanwhile, after treatment is ended and the device is withdrawn, these drugs can stay at the lesion blood vessel for a long enough time so as to play a role of preventing restenosis.

As these drugs themselves are dissolved to a certain extent in blood, it is technically challenging to deliver a sufficient dosage of the drugs to a blood vessel wall within a short treatment time (typically no more than 60 seconds) when the surface of the device is in contact with the blood vessel wall, and to still maintain an effective concentration in tissue for a longer time (e.g., 4 weeks) after operation. Meanwhile, according to records in prior literature, a drug concentration of 1 ng/mg or above in tissue 4 weeks after operation can ensure effective prevention of restenosis. Therefore, it is of great significance to provide a drug-loaded medical device that is capable of delivering a sufficient dosage of drugs to the blood vessel wall within a short treatment period when a surface of the device is in contact with the blood vessel wall, and still maintaining a certain effective concentration in tissue for a longer time after operation.

Besides, in the prior art, a preparation method for a drug-loaded medical device is often very complex, and has disadvantages such as a long process flow, waste of time and manpower and high costs.

### SUMMARY

The present invention provides a drug coat medical device, wherein this drug coat medical device can transfer enough drugs to a blood vessel wall within a time when being in contact with the blood vessel wall, and a certain drug concentration is still maintained in blood vessel wall tissue for a relatively long time after this drug coat medical device is withdrawn.

The present invention provides a preparation method for a drug coat medical device. This preparation method has advantages of simple operation, mild conditions, a short technological process, saving time, reducing costs, being suitable for mass production, etc.

The present invention provides use of the above drug coat medical device in the preparation of a drug or a medical device for treating atherosclerotic diseases, preventing restenosis and preventing local tissue proliferation.

The present invention provides a drug coat.

The present invention provides a medical device.

The present invention provides a drug coat medical device, including a carrier and a drug coat partially or completely covering the carrier.

In the above, the drug coat is mainly obtained by coating a coating liquid including a macrolide drug and a dispersant.

The dispersant includes water and/or an organic solvent, that is, only water, only an organic solvent, or both water and an organic solvent can be selected as the dispersant.

The organic solvent includes at least one of ethanol, acetone, dichloromethane, 1,4-dioxane, tetrahydrofuran and toluene.

The drug coat medical device provided in the present invention can transfer enough drugs to a blood vessel wall within a time when being in contact with the blood vessel wall, and a certain drug concentration is still maintained in blood vessel wall tissue for a relatively long time after this drug coat medical device is withdrawn.

In the above, the macrolide drug can be any conventional macrolide drug, and can be directly purchased. The macrolide drug may be in a crystalline state, or a powdered or granular form.

A system obtained by dispersing one (or more) substance(s) in another (or more) substance(s) is referred to as a dispersion system, wherein the former is a dispersed substance, called as a dispersate, and the latter plays a role of accommodating the dispersate, called as a dispersant. In the present invention, the dispersant refers to a substance playing a role of accommodating the dispersate.

Optionally, a mass ratio of the macrolide drug to the dispersant is 20~100 (25, 30, 35, 40, 45, 50, 60, 70, 80, 90 or 100 can also be selected): 100~6000 (110, 120, 150, 170, 200, 230, 250, 300, 400, 500, 600, 700, 800, 900, 1000, 1300, 1500, 1800, 2000, 2300, 2500, 2800, 3000, 4000, 5000 or 5500 can also be selected).

Using the above mass ratio is beneficial to obtaining the drug coat medical device with better performance.

Optionally, the macrolide drug includes at least one of everolimus, tacrolimus, zotarolimus, rapamycin (sirolimus), temsirolimus, biolimus, 7-O-demethylrapamycin, deforolimus, 32-deoxorapamycin and 42-O-(2-ethoxyethyl)rapamycin.

Optionally, the carrier includes a drug balloon and/or a drug eluting stent.

Optionally, the coating liquid further includes an antioxidant.

Optionally, the antioxidant includes a water-soluble antioxidant and/or a fat-soluble antioxidant.

Optionally, the water-soluble antioxidant includes at least one of malic acid, chlorogenic acid, proanthocyanidin, ascorbic acid, sodium ascorbate, calcium ascorbate, potassium ascorbate, calcium pantothenate and vitamin E polyethylene glycol succinate.

Malic acid, also known as 2-hydroxysuccinic acid, has three isomers: L-malic acid, D-malic acid and DL-malic acid. Malic acid is a colorless needle crystal or white crystalline powder, is odorless, and has a particularly pleasant sour taste.

Chlorogenic acid is an organic compound, with a chemical formula C₁₆H₁₈O₉, and has antibacterial, antiviral, leukocyte increasing, liver protecting, gallbladder benefiting, antitumor, blood pressure lowering, blood lipid reducing, free radical scavenging, central nervous system exciting and other effects.

Proanthocyanidin is a general term of a large class of polyphenolic compounds widely present in plants, and has strong effects of resisting oxidation and scavenging free radicals.

Ascorbic acid, also known as vitamin C, is a polyhydroxy compound, with a chemical formula C₆H₈O₆. It is a white crystal or crystalline powder, is odorless, has a sour taste, and gradually turns yellowish after long-term storage. It is readily soluble in water, acidic, and slightly soluble in ethanol.

A chemical formula of sodium ascorbate is C₆H₇O₆Na. It is white to yellowish white crystalline powder or particle, odorless, and slightly salty. It is readily soluble in water, and can be used as an antioxidant for food.

Calcium ascorbate is white to pale yellow crystalline powder, odorless, soluble in water, slightly soluble in ethanol, and insoluble in diethyl ether. It is more stable than Vc, and has better oxidation resistance than Vc.

Calcium pantothenate is an organic substance, with a chemical formula C₁₈H₃₂O₁₀N₂Ca, and is readily soluble in water and glycerol.

Optionally, the fat-soluble antioxidant includes at least one of vitamin E, butylated hydroxytoluene, butylated hydroxyanisole, ascorbyl palmitate, tocopherol, probucol, propyl gallate and tert-butylhydroquinone.

Vitamin E is a class of fat-soluble vitamins, including four tocopherols and four tocotrienols, and is an antioxidant.

Butylated hydroxytoluene (BHT) has anti-oxidation, anti-corrosion and other effects.

Butylated hydroxyanisole, also called as butyl hydroxy anisol, is a white to yellowish crystal or waxy solid, slightly has a special smell, and can be used as a food antioxidant.

Ascorbyl palmitate is formed by esterifying natural ingredients such as palmitic acid and L-ascorbic acid, with a chemical formula C₂₂H₃₈O₇, and is an efficient oxygen scavenger and synergist.

Tocopherol, a hydrolysis product of vitamin E, increases anti-oxidation effect of cells, maintains and promotes reproductive function, has a certain anti-aging effect, and can also improve lipid metabolism, prevent arteriosclerosis, and lower blood lipid.

Probucol is white or white-like crystalline powder, has a special odor, and has effects of regulating blood lipid and resisting lipid peroxidation.

Propyl gallate is white to yellowish-brownish crystalline powder or milky white needle crystal, and is odorless and slightly bitter. An aqueous solution thereof is tasteless. It is hygroscopic, and decomposition thereof can be promoted by light. It is poorly soluble in water and readily soluble in hot water, ethanol, diethyl ether, propylene glycol, glycerol, cotton seed oil, peanut oil, and lard.

Tert-butylhydroquinone, abbreviated as TBHQ, is a white powdered crystal and has a special smell. It is readily soluble in ethanol and ether, soluble in grease and insoluble in water.

The macrolide drug is prone to oxidation in air. Therefore, by adding the antioxidant, the present invention can improve storage stability of products, and prolong a storage validity period of the drug coat medical device.

Optionally, a mass ratio of the antioxidant to the macrolide drug is 0.01-10 (0.1, 0.5, 1, 2, 3, 4, 5, 6, 7, 8 or 9 can also be selected):1.

Optionally, when the dispersant in the coating liquid is water, the antioxidant used in the coating liquid is a water-soluble antioxidant; alternatively, when the dispersant in the coating liquid is an organic solvent, the antioxidant used in the coating liquid is a fat-soluble antioxidant.

When the dispersant in the coating liquid is both water and an organic solvent, the antioxidant used in the coating liquid may be one of a water-soluble antioxidant and a fat-soluble antioxidant, or a mixture of a water-soluble antioxidant and a fat-soluble antioxidant.

Optionally, the coating liquid further includes a polymer material.

The polymer material, on the one hand, acts as a binder (binding agent), and on the other hand, acts as a dispersion stabilizer.

In the above, the polymer material as a binder has an effect of improving adhesion between mixed materials and the carrier.

Meanwhile, the polymer material as a dispersion stabilizer enables the macrolide drug to be stably dispersed in the dispersant, which facilitates coating.

Optionally, the polymer material includes at least one of polyethylene glycol, polyethylene oxide, hyaluronic acid, carboxymethyl cellulose, collagen, polyvinyl pyrrolidone and polyvinyl alcohol.

Optionally, a mass ratio of the polymer material to the dispersant is 0.1-30 (0.5, 1, 2, 3, 4, 5, 6, 7, 8 or 9 can also be selected): 100∼1000 (120, 150, 180, 200, 250, 300, 350, 400, 450, 500, 600, 700, 800 or 900 can also be selected).

Optionally, when the dispersant in the coating liquid is an organic solvent, the drug coat is obtained by coating a coating liquid including a macrolide drug and a dispersant, and an additive solution including water together.

When water is used as the dispersant, as most of the macrolide drugs are not readily soluble in water, the coating liquid containing a macrolide drug and a dispersant is usually a suspension. In some optional embodiments of the present invention, the coating liquid is in a suspension-liquid state by means of stirring, vortexing, sonication or homogenization (using a homogenizer), etc., that is, small solid drug particles in the coating liquid are suspended in the liquid. In this way, the macrolide drug can be uniformly covered on a surface of the carrier when the coating is carried out.

When an organic solvent is used as the dispersant in the coating liquid, the drug coat is obtained by coating a coating liquid including a macrolide drug and a dispersant, and an additive solution including water together. Because the macrolide drugs are usually soluble in organic solvents, the coating liquid prepared in this case is in a solution state. By coating the additive solution including water and the coating liquid together, when the coating is carried out, the macrolide drug is separated out again when being in contact with water, and is in a crystalline, powdered or small granular form. In this way, a crystal form of the macrolide drug is not affected in a subsequent drying process.

Optionally, the additive solution further includes a water-soluble antioxidant and/or a polymer material.

Optionally, the additive solution is mainly made of following components in parts by mass: 80∼1200 parts (including but not limited to any point value of 85 parts, 90 parts, 95 parts, 100 parts, 105 parts, 110 parts, 115 parts, 200 parts, 500 parts, 800 parts, 1000 parts, 1100 parts or a range value between any two) of water, 0∼100 parts (including but not limited to any point value of 5 parts, 10 parts, 15 parts, 20 parts, 25 parts, 30 parts, 35 parts, 40 parts, 45 parts, 50 parts, 60 parts, 70 parts, 80 parts, 90 parts or a range value between any two) of a water-soluble antioxidant and 0~300 parts (including but not limited to any point value of 10 parts, 30 parts, 50 parts, 80 parts, 100 parts, 130 parts, 150 parts, 180 parts, 200 parts, 230 parts, 250 parts, 270 parts, 290 parts or a range value between any two) of a polymer material.

Optionally, a mass ratio of the coating liquid to the additive solution is 1∼7 (2, 3, 4, 5 or 6 can also be selected): 1∼7 (2, 3, 4, 5 or 6 can also be selected).

In some optional embodiments of the present invention, a coating concentration of the coating liquid, based on the macrolide drug, is 0.05 µg/mm²~50 µg/mm², including but not limited to any point value of 0.1 µg/mm², 0.5 µg/mm², 1 µg/mm², 3 µg/mm², 5 µg/mm², 8 µg/mm², 10 µg/mm², 15 µg/mm², 20 µg/mm² , 25 µg/mm², 30 µg/mm², 35 µg/mm² , 40 µg/mm², 45 µg/mm² or a range value between any two.

The present invention further provides a preparation method for the above drug coat medical device, including a step of:

coating a coating liquid including a macrolide drug and a dispersant on a surface of the carrier to obtain the drug coat medical device.

The preparation method provided in the present invention is simple and easy to implement, has a short process flow and mild conditions, not only can save a lot of time, but also can reduce costs, and is also suitable for mass production.

Optionally, the present invention is to directly coat the drug on the surface of the carrier in a form of small particles.

In the prior art, the preparation method for a drug coat medical device is often very complex, for example, hydrophilic pre-treatment needs to be performed on the carrier, or a given material is pre-coated on the surface of the carrier to render a priming layer, and for another example, a drug crystal is directly grown on the carrier.

However, the preparation method provided in the present invention is quite simple to operate, has a short process flow, and consumes a short time. Moreover, the drug coat medical device prepared by this method can transfer enough drugs to the blood vessel wall, and a certain drug concentration is still maintained in the blood vessel wall tissue for a relatively long time after the drug coat medical device is withdrawn. Optionally, the drug concentration in tissue is still 1 ng/mg or above 4 weeks after operation, which can meet requirements in the medical field.

Optionally, a D50 particle size of the macrolide drug is less than 200 µm, including but not limited to any point value one of 190 µm, 180 µm, 170 µm, 160 µm, 150 µm, 130 µm, 110 µm, 100 µm, 80 µm, 60 µm, 50 µm, 30 µm, 20 µm, 10 µm, 5 µm, 3 µm, 1 µm, 0.5 µm, 0.3 µm, 0.1 µm, 0.05 µm or a range value between any two.

Optionally, the coating concentration of the coating liquid, based on the drug, is 0.05 µg/mm²~50 µg/mm², including but not limited to any point value of 0.1 µg/mm², 0.5 µg/mm², 1 µg/mm² , 3 µg/mm² , 5 µg/mm², 8 µg/mm², 10 µg/mm², 15 µg/mm², 20 µg/mm², 25 µg/mm², 30 µg/mm², 35 µg/mm², 40 µg/mm², 45 µg/mm² or a range value between any two.

Optionally, a method of the coating includes at least one of spray coating, dip coating, drop coating and brush coating.

Optionally, the spray coating includes ultrasonic spray coating.

Ultrasonic spray coating, also called as ultrasonic-wave spray coating, refers to a spray-coating process using an ultrasonic atomization technology. A material spray-coated thereby may be a solution or a suspension. The ultrasonic spray coating has advantages such as high uniformity of a coating, high utilization rate of raw materials, less splashing, and easy control of a spray-coating amount.

Optionally, after the coating, the method further includes steps of drying, packaging and sterilizing in sequence.

Optionally, when the dispersant in the coating liquid is an organic solvent, the method of coating includes a step of:

simultaneously coating a coating liquid including a macrolide drug and a dispersant and an additive solution including water to the surface of the carrier.

Optionally, the additive solution further includes a water-soluble antioxidant and/or a polymer material. In the above, the water-soluble antioxidant includes at least one of malic acid, chlorogenic acid, proanthocyanidin, ascorbic acid, sodium ascorbate, calcium ascorbate, potassium ascorbate, calcium pantothenate and vitamin E polyethylene glycol succinate; and the polymer material includes at least one of polyethylene glycol, polyethylene oxide, hyaluronic acid, carboxymethyl cellulose, collagen, polyvinyl pyrrolidone and polyvinyl alcohol.

In the present invention, simultaneous coating means that the coating liquid and the additive solution are simultaneously coated on the surface of the carrier, and the coating liquid and the additive solution are coated at the same position. When the spray coating is used, the coating liquid and the additive solution are simultaneously spray-coated onto the surface of the carrier from two directions, and form cross points on the surface of the carrier during the spray coating. In this way, the macrolide drug is separated out again when being in contact with water, and thus the crystal form of the macrolide drug will not be affected in a subsequent drying process, thus being favorable for ensuring drug effect.

Optionally, a temperature of the coating liquid and/or the additive solution is lower than 70 °C, including but not limited to any point value of 60 °C, 50 °C, 40 °C, 30 °C, 20 °C, 10 °C, 5 °C or a value range between any two.

The present invention further provides use of the above drug coat medical device, or the drug coat medical device prepared by the above preparation method for a drug coat medical device in the preparation of a drug or a medical device for treating atherosclerotic diseases, preventing restenosis and preventing local tissue proliferation.

The present invention further provides a drug coat, wherein the drug coat consists of a macrolide drug and a water-soluble antioxidant.

Optionally, the macrolide drug includes at least one of everolimus, tacrolimus, zotarolimus, rapamycin, temsirolimus, biolimus, 7-O-demethylrapamycin, deforolimus, 32-deoxorapamycin and 42-O-(2-ethoxyethyl)rapamycin.

Optionally, the water-soluble antioxidant includes at least one of malic acid, chlorogenic acid, proanthocyanidin, ascorbic acid, sodium ascorbate, calcium ascorbate, potassium ascorbate, calcium pantothenate and vitamin E polyethylene glycol succinate.

Optionally, a mass ratio of the water-soluble antioxidant to the macrolide drug is 0.01-10 (0.1, 0.5, 1, 2, 3, 4, 5, 6, 7, 8 or 9 can also be selected):1.

The present invention further provides a medical device, including the above drug coat.

Compared with the prior art, the present invention has following beneficial effects:
(1) The drug coat medical device provided in the present invention can transfer enough drugs to the blood vessel wall when being in contact with the blood vessel wall, and a certain drug concentration is still maintained in the blood vessel wall tissue for a relatively long time after the drug coat medical device is withdrawn.
(2) The preparation method for a drug coat medical device provided in the present invention has advantages of simple operation, mild conditions, a short technological process, saving time, reducing costs, being suitable for mass production, etc.
(3) In the preparation method for a drug coat medical device provided in the present invention, by simultaneously coating the coating liquid including the macrolide drug and the dispersant and the additive solution including water to the surface of the carrier, on the premise of greatly simplifying a preparation process, a relatively high drug concentration still can be maintained in the blood vessel wall tissue for a relatively long time after the drug coat medical device is withdrawn.

### BRIEF DESCRIPTION OF DRAWINGS

In order to more clearly illustrate the technical solutions in embodiments of the present invention or in the prior art, drawings which need to be used in the description of the embodiments or the prior art will be introduced briefly below. Apparently, the drawings in the description below merely show some embodiments of the present invention, and those ordinarily skilled in the art still could obtain other drawings in light of these drawings without using any inventive efforts.
FIG. 1 is an SEM image of a drug coat medical device provided in Example 1 of the present invention; and
FIG. 2 is an SEM image of the drug coat medical device provided in Example 2 of the present invention.

### DETAILED DESCRIPTION OF EMBODIMENTS

Technical solutions of the present invention will be described clearly and completely below in combination with the embodiments, while those skilled in the art would understand that the embodiments described below are some but not all embodiments of the present invention, and they are merely used for illustrating the present invention, but should not be considered as limiting the scope of the present invention. Based on the examples in the present invention, all of other examples obtained by those ordinarily skilled in the art without using any inventive efforts shall fall within the scope of protection of the present invention. Examples, for which no concrete conditions are specified, are performed according to conventional conditions or conditions recommended by the manufactures. Where manufacturers of reagents or apparatuses used are not specified, they are conventional products commercially available.

In an embodiment of the present invention, the preparation method for a drug coat medical device includes a step of:
coating a suspension liquid (suspension) including a macrolide drug and water on a surface of a carrier, to obtain a drug coat medical device.

Optionally, the suspension liquid further includes a water-soluble antioxidant and/or a polymer material.

In another embodiment of the present invention, the preparation method for a drug coat medical device includes a step of:
simultaneously coating a mixed solution containing a macrolide drug and an organic solvent, and an additive solution containing water on a surface of a carrier, and making coating areas of the mixed solution and the additive solution coincide, to obtain the drug coat medical device.

Optionally, the mixed solution further includes a fat-soluble antioxidant.

Optionally, the additive solution further includes a water-soluble antioxidant and/or a polymer material.

Sirolimus, everolimus, and tacrolimus used in following examples and comparative examples of the present invention are all purchased.

### Example

### Example 1

A preparation method for a drug coat medical device provided in the present example includes following steps.

To 1000 mg of distilled water, 30 mg of sirolimus (with a D50 particle size less than 200 µm, and manufactured by Huabei Pharmaceuticals) was added, to form a stable suspension by ultrasonic oscillation (a temperature of the suspension was 20 °C). Then the above suspension was ultrasonically spray-coated on surfaces of drug balloons, with a drug concentration on the surfaces of the drug balloons being 4 µg/mm². After drying, the drug coat medical device was obtained.

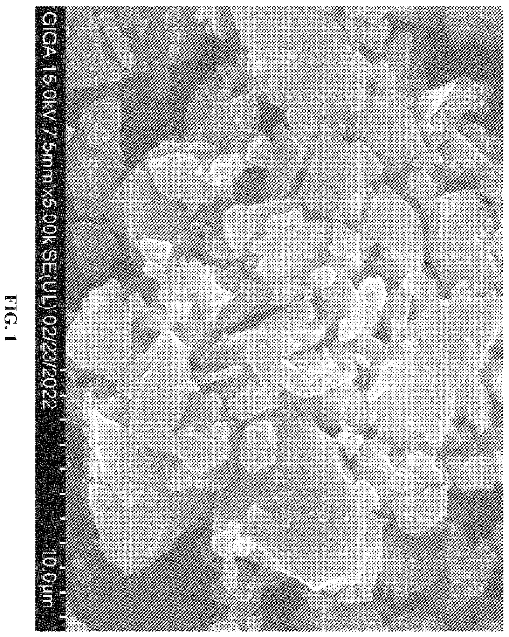

SEM detection was performed on the drug coat medical device, and test results are as shown in FIG. 1. It can be seen from FIG. 1 that the drug is distributed in a granular form on a surface of the device.

### Example 2

A preparation method for a drug coat medical device provided in the present example includes following steps.

To 1000 mg of distilled water, 30 mg of ascorbic acid was added and stirred to be fully dissolved, and then 25 mg of sirolimus (with a D50 particle size less than 200 µm, and manufactured by NEWBIO PHARM-TECH) was added thereto. Mixture was stirred at a high speed using a homogenizer to form a suspension (a temperature of the suspension was 25 °C), and the suspension was oscillated in an ultrasonic cleaner to keep it in a stable suspension state.

The above suspension was brush-coated on surfaces of drug balloons, with a drug concentration on the surfaces of the drug balloons being 3 µg/mm². After drying, the drug coat medical device was obtained.

That is, a drug coat was also obtained in the present example, and this drug coat consisted of a macrolide drug (sirolimus) and ascorbic acid.

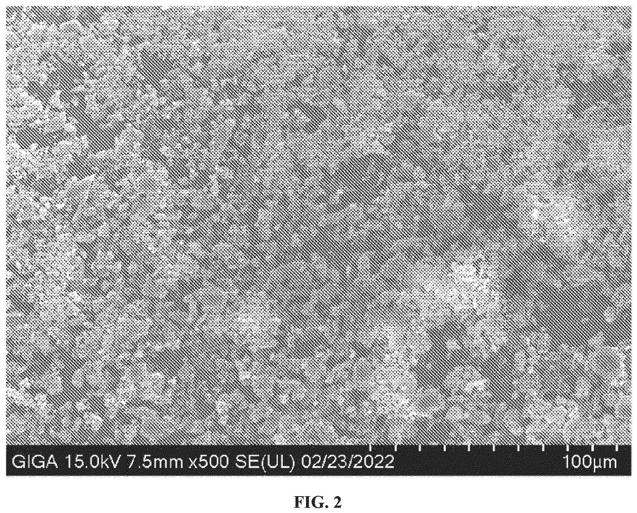

SEM detection was performed on the drug coat medical device, and test results are as shown in FIG. 2. It can be seen from FIG. 2 that the drug is distributed in an obvious granular form on a surface of the device.

### Example 3

A preparation method for a drug coat medical device provided in the present example includes following steps.

30 mg of sirolimus (with a D50 particle size less than 200 µm, and manufactured by Huadong Medicine Co., Ltd) was weighed and dissolved in 1000 mg of ethanol, to render a coating liquid (with a temperature of 25 °C).

To 1000 mg of distilled water, 50 mg of L-malic acid was added and stirred to be fully dissolved, to render an additive solution (with a temperature of 25 °C).

The above coating liquid and additive solution were simultaneously ultrasonically spray-coated on surfaces of drug balloons to form a white powdered coating, with a drug concentration of sirolimus on the surfaces of the drug balloons being 2.5 µg/mm², and a concentration of L-malic acid being 1.3 µg/mm². After drying, the drug coat medical device was obtained.

That is, a drug coat was also obtained in the present example, and this drug coat consisted of a macrolide drug (sirolimus) and L-malic acid.

### Example 4

A preparation method for a drug coat medical device provided in the present example includes following steps.

To 1000 mg of distilled water, 25 mg of polyethylene glycol (with a molecular weight being 20000) was added and stirred to be fully dissolved, and then 25 mg of sirolimus (with a D50 particle size less than 200 µm, and manufactured by Hisun Pharmaceutical Co., Ltd.) was added thereto. Mixture was stirred at a high speed using a homogenizer to form a stable suspension (a temperature of the suspension was 30 °C). The above suspension was ultrasonically spray-coated on surfaces of drug balloons, with a drug concentration on the surfaces of the drug balloons being 1 µg/mm². After drying, the drug coat medical device was obtained.

### Example 5

A preparation method for a drug coat medical device provided in the present example includes following steps.

30 mg of sirolimus (with a D50 particle size less than 200 µm, and manufactured by Xinchang Pharmaceutical Factory) was weighed and dissolved in 5000 mg of acetone, to render a coating liquid (with a temperature of 35 °C).

To 1000 mg of distilled water, 30 mg of calcium pantothenate was added and stirred to be fully dissolved, to render an additive solution (with a temperature of 35 °C).

The above coating liquid and additive solution were simultaneously ultrasonically spray-coated on surfaces of drug balloons to form a white powdered coating, with a drug concentration of sirolimus on the surfaces of the drug balloons being 5 µg/mm², and a concentration of L-malic acid being 4.8 µg/mm². After drying, the drug coat medical device was obtained.

### Example 6

A preparation method for a drug coat medical device provided in the present example includes following steps.

To 3000 mg of distilled water, 30 mg of everolimus (with a D50 particle size less than 200 µm, and manufactured by Hubei Jiuzhou Kangda Biotechnology Co., Ltd) was added, to form a stable suspension by ultrasonic oscillation (a temperature of the suspension was 65 °C). Then the drug balloons were immersed in the above suspension, with a drug concentration of everolimus on the surfaces of the drug balloons being 10 µg/mm². After drying, packaging and sterilization in sequence, the drug coat medical device was obtained.

### Example 7

A preparation method for a drug coat medical device provided in the present example includes following steps.

To 1000 mg of distilled water, 100 mg of everolimus (with a D50 particle size less than 200 µm, and manufactured by WUHAN BIOCAR BIO-PHARM CO., LTD.), 1 mg of hyaluronic acid and 300 mg of sodium ascorbate were added, to form a stable suspension by ultrasonic oscillation (a temperature of the suspension was 40 °C). Then the above suspension was ultrasonically spray-coated on a surface of a drug eluting stent, with a drug concentration on the surface of the drug eluting stent being 50 µg/mm². After drying, packaging and sterilization in sequence, the drug coat medical device was obtained.

### Example 8

A preparation method for a drug coat medical device provided in the present example includes following steps.

30 mg of tacrolimus (with a D50 particle size less than 200 µm, and manufactured by Jinan Jia Ge Biotechnology Co., Ltd) was weighed and dissolved in 100 mg of dichloromethane, and then 150 mg of vitamin E was added thereto, to render a coating liquid (with a temperature of 20 °C).

The above coating liquid and 200 mg of distilled water (i.e., additive solution) were simultaneously ultrasonically spray-coated on a surface of a drug eluting stent, to form a white powdered coating, with a drug concentration of sirolimus on the surface of the drug eluting stent reaching 8 µg/mm². After drying, the drug coat medical device was obtained.

### Example 9

A preparation method for a drug coat medical device provided in the present example includes following steps.

100 mg of tacrolimus (with a D50 particle size less than 200 µm, and manufactured by Hisun Pharmaceutical Co., Ltd.) was weighed and dissolved in 1000 mg of methylbenzene, to render a coating liquid (with a temperature of 25 °C).

To 1000 mg of distilled water, 100 mg of L-malic acid and 250 mg of carboxymethyl cellulose were added and stirred to be fully dissolved, to render an additive solution (with a temperature of 25 °C).

The above coating liquid and additive solution were simultaneously ultrasonically spray-coated on surfaces of drug balloons to form a white powdered coating, with a drug concentration of sirolimus on the surfaces of the drug balloons being 2 µg/mm², a concentration of L-malic acid being 1 µg/mm², and a concentration of carboxymethyl cellulose being 3 µg/mm² . After drying, the drug coat medical device was obtained.

### Example 10

A preparation method for a drug coat medical device provided in the present example includes following steps.

To 1000 mg of distilled water, 30 mg of chlorogenic acid was added and stirred to be fully dissolved, and then 25 mg of rapamycin (with a D50 particle size less than 200 µm, and manufactured by Yibin Wish Pharmaceutical Co., Ltd) was added thereto. Mixture was stirred at a high speed using a homogenizer to form a suspension (a temperature of the suspension was 25 °C). The suspension was oscillated in an ultrasonic cleaner to keep it in a stable suspension state.

The above suspension was brush-coated on surfaces of drug balloons, with a drug concentration on the surfaces of the drug balloons being 10 µg/mm². After drying, the drug coat medical device was obtained.

That is, a drug coat was also obtained in the present example, and this drug coat consisted of a macrolide drug (rapamycin) and chlorogenic acid.

### Comparative Example 1

A preparation method for a drug coat medical device provided by the present comparative example is substantially the same as that in Example 1, and is merely different in that distilled water was replaced with ethanol of equal mass.

### Comparative Example 2

A preparation method for a drug coat medical device provided by the present comparative example is substantially the same as that in Example 1, and is merely different in that the D50 particle size of sirolimus was changed to be greater than 200 µm.

### Comparative Example 3

A preparation method for a drug coat medical device provided by the present comparative example is substantially the same as that in Example 1, and is merely different in that mass of sirolimus was changed to be 100 mg.

### Comparative Example 4

A preparation method for a drug coat medical device provided by the present comparative example is substantially the same as that in Example 3, and is merely different in that the step "The above coating liquid and additive solution were simultaneously ultrasonically spray-coated on surfaces of drug balloons" in Example 3 was replaced with "the above coating liquid and additive solution were well mixed and then ultrasonically spray-coated on surfaces of drug balloons".

### Experimental Example

The drug coat medical devices prepared by the above examples and comparative examples were used to perform animal experiment, by a following method: the drug coat medical devices were guided into coronary arteries of pigs by DSA radiography (digital subtraction angiography), the coronary arteries were expanded with 6 atmospheres for 60 seconds and then depressurized, and the drug coat medical devices were withdrawn. After being fed for 4 weeks, the experimental animals were sacrificed, the coronary arteries thereof were taken and weighed, homogenized and extracted, and drug concentrations in tissue were measured with liquid chromatography-mass spectrometry. Results are as shown in TABLE 1 below.

**TABLE 1 Results of Drug Concentrations in Tissues in Groups**

| Group | Drug Concentration in Tissue (ng/mg) |
|---|---|
| Example 1 | 1.25±0.81 |
| Example 2 | 2.90±1.91 |
| Example 3 | 6.09±4.78 |
| Example 4 | 2.05±2.01 |
| Example 5 | 5.30±4.79 |
| Example 6 | 2.3±1.8 |
| Example 7 | 3.6±2.1 |
| Example 8 | 1.9±1.2 |
| Example 9 | 3.1±1.8 |
| Example 10 | 4.2±3.8 |
| Comparative Example 1 | Below limit of detection |
| Comparative Example 2 | Below limit of detection |
| Comparative Example 3 | 1.51±1.01 |
| Comparative Example 4 | Below limit of detection |

As can be seen from TABLE 1, the drug concentrations in tissues in respective examples of the present invention are all 1 ng/mg or above. Therefore, prevention of restenosis can be ensured.

Although the present invention has been illustrated and described with examples, it should be realized that the various examples above are merely used for illustrating the technical solutions of the present invention, rather than limiting the present invention; those ordinarily skilled in the art should understand that, without departing from the spirit and scope of the present invention, they still could modify the technical solutions described in various preceding examples, or make equivalent substitutions to some or all of the technical features therein; and these modifications or substitutions do not make corresponding technical solutions essentially depart from the scope of the technical solutions of various examples of the present invention; therefore, it means that the attached claims cover all of these substitutions and modifications within the scope of the present invention.

## Claims

1. A drug coat medical device, comprising a carrier and a drug coat partially or completely covering the carrier,
wherein the drug coat is mainly obtained by coating a coating liquid comprising a macrolide drug and a dispersant; and
the dispersant comprises water and/or an organic solvent, wherein the organic solvent comprises at least one of ethanol, acetone, dichloromethane, 1,4-dioxane, tetrahydrofuran and toluene.

2. The drug coat medical device according to claim 1, wherein a mass ratio of the macrolide drug to the dispersant is 20~100:100~6000;
preferably, the macrolide drug comprises at least one of everolimus, tacrolimus, zotarolimus, rapamycin, temsirolimus, biolimus, 7-O-demethylrapamycin, deforolimus, 32-deoxorapamycin and 42-O-(2-ethoxyethyl)rapamycin; and
preferably, the carrier comprises a drug balloon and/or a drug eluting stent.

3. The drug coat medical device according to claim 1, wherein the coating liquid further comprises an antioxidant;
preferably, the antioxidant comprises a water-soluble antioxidant and/or a fat-soluble antioxidant;
preferably, the water-soluble antioxidant comprises at least one of malic acid, chlorogenic acid, proanthocyanidin, ascorbic acid, sodium ascorbate, calcium ascorbate, potassium ascorbate, calcium pantothenate and vitamin E polyethylene glycol succinate;
preferably, the fat-soluble antioxidant comprises at least one of vitamin E, butylated hydroxytoluene, butylated hydroxyanisole, ascorbyl palmitate, tocopherol, probucol, propyl gallate and tert-butylhydroquinone;
preferably, a mass ratio of the antioxidant to the macrolide drug is 0.01∼10:1; and
preferably, when the dispersant in the coating liquid is water, the antioxidant used in the coating liquid is the water-soluble antioxidant; or when the dispersant in the coating liquid is the organic solvent, the antioxidant used in the coating liquid is the fat-soluble antioxidant.

4. The drug coat medical device according to claim 1, wherein the coating liquid further comprises a polymer material;
preferably, the polymer material comprises at least one of polyethylene glycol, polyethylene oxide, hyaluronic acid, carboxymethyl cellulose, collagen, polyvinyl pyrrolidone and polyvinyl alcohol; and
preferably, a mass ratio of the polymer material to the dispersant is 0.1-30:100-1000.

5. The drug coat medical device according to any one of claims 1-4, wherein when the dispersant in the coating liquid is the organic solvent, the drug coat is obtained by coating the coating liquid comprising the macrolide drug and the dispersant, and an additive solution comprising water together.

6. The drug coat medical device according to claim 5, wherein the additive solution further comprises a water-soluble antioxidant and/or a polymer material; and
preferably, the additive solution is mainly made of following components in parts by mass: 80∼1200 parts of water, 0~100 parts of the water-soluble antioxidant and 0~300 parts of the polymer material.

7. A preparation method for the drug coat medical device according to any one of claims 1-6, comprising a step of:
coating the coating liquid comprising the macrolide drug and the dispersant on a surface of the carrier to obtain the drug coat medical device.

8. The preparation method according to claim 7, wherein a D50 particle size of the macrolide drug is less than 200 µm;
preferably, a coating concentration of the coating liquid, based on the macrolide drug, is 0.05 µg/mm²~50 µg/mm²;
preferably, a method of the coating comprises at least one of spray coating, dip coating, drop coating and brush coating; and
preferably, after the coating, the preparation method further comprises steps of drying, packaging and sterilizing in sequence.

9. The preparation method according to claim 7, wherein the method of the coating further comprises a step of:
simultaneously coating the coating liquid comprising the macrolide drug and the dispersant and an additive solution comprising water to the surface of the carrier; and
preferably, a temperature of the coating liquid and/or the additive solution is lower than 70 °C.

10. Use of the drug coat medical device according to any one of claims 1~6, or the drug coat medical device prepared by the preparation method for the drug coat medical device according to any one of claims 7∼9 in a preparation of a drug or a medical device for treating atherosclerotic diseases, preventing restenosis and preventing local tissue proliferation.

11. A drug coat, wherein the drug coat consists of a macrolide drug and a water-soluble antioxidant.

12. The drug coat according to claim 11, wherein the macrolide drug comprises at least one of everolimus, tacrolimus, zotarolimus, rapamycin, temsirolimus, biolimus, 7-O-demethylrapamycin, deforolimus, 32-deoxorapamycin and 42-O-(2-ethoxyethyl)rapamycin;
preferably, the water-soluble antioxidant comprises at least one of malic acid, chlorogenic acid, proanthocyanidin, ascorbic acid, sodium ascorbate, calcium ascorbate, potassium ascorbate, calcium pantothenate and vitamin E polyethylene glycol succinate; and
preferably, a mass ratio of the water-soluble antioxidant to the macrolide drug is 0.01~10:1.

13. A medical device, comprising the drug coat according to claim 11 or 12.
